(19)

Europäisches Patentamt

European Patent Office

Office européen des brevets

(11) **EP 2 149 414 A1**

(12) **EUROPEAN PATENT APPLICATION**

(43) Date of publication:
**03.02.2010 Bulletin 2010/05**

(51) Int Cl.:
*B22F 3/11* (2006.01)          *C22C 1/08* (2006.01)
*A61L 27/04* (2006.01)          *A61L 27/42* (2006.01)
*A61L 27/54* (2006.01)          *A61L 27/56* (2006.01)

(21) Application number: **08161449.7**

(22) Date of filing: **30.07.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Nederlandse Centrale Organisatie Voor Toegepast Natuurwetenschappelijk Onderzoek TNO 2628 VK Delft (NL)**

(72) Inventors:
• **Sillekens, Wilhelmus Hubertina 6049 HA Herten (NL)**

• **Erinc, Müge 5641 GB Eindhoven (NL)**
• **Werkhoven, Robert Jan 5671 DH Nuenen (NL)**
• **Korbee, Roland Alexander 6133 VR Sittard (NL)**
• **Toonssen, Petrus Johannes 5258 JC Berlicum (NL)**

(74) Representative: **Hatzmann, Martin Vereenigde Johan de Wittlaan 7 2517 JR Den Haag (NL)**

(54) **Method of manufacturing a porous magnesium, or magnesium alloy, biomedical implant or medical appliance.**

(57)     Method of manufacturing a porous metal or metal alloy structure having a predefined porosity for use as a biomedical implant or medical appliance. The method includes: mixing predefined amounts of a magnesium or magnesium alloy powder and a binder powder into a flowable powder mixture; providing a mould and injecting the flowable powder mixture into the mould to obtain a preform moulded into a desired shape. At least the binder is then allowed to solidify. Further the binder is removed from the preform to obtain a substantially binder-free preform. The substantially binder-free preform is then sintered during a time and at a temperature to achieve the predefined porosity. The method according to the invention can be further improved in that the magnesium or magnesium alloy structure is further provided with at least one substance that adds a predetermined functionality thereto.

EP 2 149 414 A1

**Description**

The invention relates to a method of manufacturing an open porous metallic structure. More in particular the invention concerns a method of obtaining a magnesium, or magnesium alloy, biomedical implant or medical appliance.

Several techniques are available to produce an open porous metallic structure. Porous magnesium articles suitable for biomaterial, such as implants, or medical appliances, have been prepared by some specific methods. A first technique comprises of foaming by injection of argon gas into molten magnesium; yet, this does not always produce a product with consistent morphology. A second known technique is a plaster casting method using polyurethane foam; however, compressive strength of the structure does not always suffice for load-bearing applications due to the large pore size. A third technique is a powder metallurgy technique using space-holding particles; a drawback of this method is that it is not very well suited for series production of finished products.

Banhart J.; "Manufacture, characterisation and application of cellular metals and metal foams"; Progress in Materials Science 46 (2001): pages 559-632, reviews several manufacturing techniques for obtaining such metal foams and other porous metallic structures. In this publication the manufacturing techniques are classified according to the state of matter in which the metal is processed, such as solid, liquid, gaseous or ionized. In US 6725901 a method is disclosed of making porous medical devices of biocompatible metal or metal alloys. Porosity is obtained by mixing of a dissolvable, sublimable or vaporizable binder, which can later be removed. A porous stent, obtained by this method, may be impregnated with a drug releasing agent or other pharmacologic compounds. United States published patent application US 2006-0271168 also discloses an implantable medical device that degrades upon contact with body fluids and thereby limits its residence time within the body of a patient. The device is formed of a foamed porous metal that simultaneously offers high strength and an accelerated corrosion rate. Another effort in preparing a surgical implement of porous ceramic material is disclosed in US 6641776. In WO2007068479 the disclosed invention relates to a material for fixing bone fractures and/or damage, using a material that contains a mixture of apatite and a magnesium alloy. It uses grinding of both constituents in a ball grinder to obtain a homogeneous mixture which is subsequently consolidated by extrusion, forging or the like. This method does not result in porous structures. While the prior art has been striving to obtain several desirous features, none has so far been successful in offering these in an appropriate combination and without drawbacks. Firstly the previously referred to solutions often do not result in a product with consistent morphology. Secondly compressive strength of the structures obtained does not always suffice for load-bearing applications due to their relatively large pore sizes. And, thirdly, the prior art methods are not very well suited for series production of finished products. Moreover none of these known techniques seems at present to be in industrial use.

Accordingly it is an object of the present invention to overcome or ameliorate at least one of the disadvantages of the prior art. It is also an object of the present invention to provide alternative solutions which are less cumbersome in performing and operation and which moreover can be practised relatively inexpensively. Alternatively it is an object of the invention to at least provide the public with a useful choice.

To this end the invention provides for a method of manufacturing a porous metal or metal alloy structure having a predefined porosity, the method including: mixing predefined amounts of a metal or metal alloy powder and a binder powder into a flowable powder mixture; providing a mould and injecting the flowable powder mixture into the mould to obtain a preform moulded into a desired shape; allowing solidification of at least the binder; removing the binder from the preform to obtain a substantially binder-free preform; sintering the substantially binder-free preform during a time and at a temperature to achieve the redefined porosity, and wherein the mixing is performed using metal or metal alloy powder that includes magnesium or at least one of its alloys.

Conventional metal injection moulding (MIM) is generally used for relatively complex parts made of high-strength and hard metal alloys, such as Fe-Ni steels, stainless steels, copper, Fe-Co magnetic alloys, Fe-Ni alloys, nickel based super alloys or tungsten heavy alloys. Metal injection moulding has not been used for magnesium or magnesium alloys, because die casting is available for low-temperature alloys, such as zinc, aluminium, or magnesium. Magnesium and magnesium alloys were thus not of interest for traditional MIM applications. Porosity in connection with magnesium and its alloys is generally regarded unfavourable as it compromises the mechanical characteristics of the part and has so far been regarded as something to be prevented. Nonetheless the inventors have discovered that metal injection moulding of magnesium and its alloys when carried out properly can provide the appropriate combination of desirable features in porous structures for medical appliances or implants.

According to a further aspect of the invention the binder can be a polymeric material, preferably a thermoplastic binder. It is further preferred when the polymeric binder is a high molecular weight compound, such as polypropylene (PP). The high molecular weight compound can also be selected from a group comprising polyethylene, methacrylate ester copolymers, and ethylene-vinyl acetate copolymer. In a particular variation the binder can also be a biodegradable polymer and this may preferably be selected from a group comprising poly-L-lactic (PLLA), polyglycolic acid (PGA), poly DL-lactide (PDLA), poly DL-lactide/glycolide (PGLA), and polyprolactine (PCL).

According to another variation of the invention the binder can be mixed with wax and the wax may preferably be selected from a group comprising paraffin wax, carnauba wax, and other low molecular weight compounds. According to a still further aspect of the invention the metal or metal alloy structure obtained by the method is a medical appliance, such as a biomedical implant.

In a particular advantageous variation of the method according to the invention the binder is removed with a solvent, preferably by solvent immersion. In another advantageous variation of the method according to the invention the binder can be removed thermally. This can be accomplished by heating the moulded preform gradually in one of a controlled atmosphere and vacuum to allow the binder to evaporate. The controlled atmosphere thereby can be an inert environment in which the binder is chemically decomposed at a temperature between 300 and 350 °C. The method according to the invention can be further improved in that the metal or metal alloy structure is further provided with at least one substance that adds a predetermined functionality thereto. This can be done in that at the mixing step the powder mixture is charged with the at least one substance, as an additive that is left behind in the metal structure after debinding. Further it can thereby be advantageous when a polymer binder is charged with at least one, preferably bio-active, substance that is left behind in the metal structure after debinding. For this it is preferable when the at least one substance remains stable up to approximately 600 degrees Celsius (°C). A suitable substance can then be hydroxyl-apatite (HA). Also it is possible for the at least one substance to be contained in a coating, which is applied to, or in, the porous metal or metal alloy structure. In the latter case the at least one substance may also be added after the porous structure itself is finished. This can be accomplished by immersion of the finished porous structure in a fluid containing the substance.

Similarly it can be advantageous when anti-inflammatory drugs are added that disintegrate at higher temperatures. This may be a bio-active substance, which includes an anti-inflammatory agent. The anti-inflammatory agent in particular may be selected from a group comprising glucocorticoids, betamethasone, dexamethasone, prednisolone, corticosterone, budesonide, estrogen, sulfasalazine, and mesalamine. The bio-active substance may alternatively also include an immuno-suppressive drug and this may be selected from a group comprising analogues of rapamycin, including tacrolimus (FK-506), sirolimus and preferably everolimus. The immuno-suppressive drug may also be selected from a group comprising morpholino oligos, used in antisense knockdown of c-myc, other medication against restenosis, paclitaxel, abraxane®, and docetaxel. It is further optionally possible for the bio-active substance to include hydroxyl-apatite.

According to yet another aspect of the invention morphology of the porous metal or metal alloy structure is preferably controlled so as to provide mechanical and desorption properties useful for use of the obtained structure as an implant. In this connection it is advantageous to control the morphology by appropriate selection of metal powders, as to their size, shape and distribution. In particular powders for the powder mixture can be selected with a narrow range of particle sizes, preferably with the particle size selected to be between 3 to 100 microns. The morphology can be further controlled by appropriate selection of blending ratios, as to fractions of metal and polymer. As a non-limiting example the metal powder can be present in a ratio of 60-65% by volume and the binder powder in a ratio of 40-35% by volume. Further the morphology can be controlled by appropriate selection of sintering parameters, as to temperature, and time. More specifically sintering can be effected at a temperature between 350 and 550 °C, for a duration of between 20 and 60 minutes.

The method according to the invention can advantageously be implemented to achieve ranges of porosity and pore size that are targeted for biomedical implants and comprise a relative density of 60 to 100% and a pore size of 0.1 mm or less.

In a further advantageous implementation of the method according to the invention mechanical and desorption properties of structures obtained thereby can be tuned by selecting amongst different magnesium alloy compositions for powder alloys and powder mixtures. In particular such tuning of the mechanical and desorption properties can include presence and amount of major and minor alloying elements. The alloying elements can thereby preferably including any one of aluminium, zinc, and manganese.

In general the method according to the invention may use alloying elements that are bio-compatible.

It is also advantageous for the method according to the invention when the steps of binder removal after moulding and sintering are combined into one single thermal operation by using a binder that has a debinding temperature close to, or equalling, the sintering temperature. In that case the debinding temperature of the binder may range from about 350 to 550 °C.

The method of the invention may use a biodegradable polymer as a binder and a given amount of the binder may then intentionally be left in the metal or metal alloy structure to obtain predefined mechanical properties. According to yet another aspect of the invention at least one substance may be added that serves as a corrosion inhibitor. It is also possible for such an added substance to serve in adding a different functionality and in particular it may serve as a stimulator of bone growth. Such a substance may be hydroxyl-apatite: $Ca_{10}(PO_4)_6(OH)_2$.

The method according to the invention may further advantageously include a subsequent coating step so as to provide any one of a desorption time and addition of other functionality to the open porous metal or metal alloy structure. In particular a plasma electric oxidation (PEO) step, known as Keronite process, may be used to enable penetration of pores and provide internal surfaces thereof with any one of a corrosion-retarding coating and a bio-active substances, such as preferably hydroxyl-apatite.

It is also advantageous for the method according to the invention when prior to the step of injecting into the mould, an insert of a different metal is positioned in the mould to manufacture a hybrid structure offering differentiated functionalities. In this regard the hybrid structure that is obtained may be partly resorbable and partly non-resorbable. The insert may advantageously be of a metal or metal alloy selected from a group including Titanium alloys, such as Ti-6Al-4V, Co-Cr-Mo, AISI316L, Ta-based, pure iron (Fe), Fe-Mn alloys, Mg alloys and pure Mg.

In the method according to the invention, the mixing of the magnesium based powders and the binder powders may use a chaotic mixing process for improving product homogeneity.

In the method according to the invention, metal injection moulding may be implemented in a continuous fashion by moulding on a roller, so as to manufacture a sheet-like preform as a basis for a porous magnesium or magnesium alloy sheet.

The invention enables the manufacturing of open porous magnesium alloy structures of relatively high density with controlled and consistent pore morphology and including any supplementary substances and/or coatings. These structures are to serve as biomedical implants, where the porosity and its inclusions facilitate the healing process while the magnesium is resorbed by the body at a similar pace. The inclusion of any supplementary substances to tune the behaviour of the part is directly connected to the intended porous structure. There is currently no appropriate manufacturing process for such biomedical implants. The process according to the invention is to use magnesium alloy powders/granules in combination with a polymer and any supplementary substances, where the sintering process targets the realization of a structure of a pre-defined porosity rather than a fully dense compound. Benefits over the current solution will now be listed.

The process according to the invention can be used to manufacture resorbable implants that have to provide initial strength and stiffness but become redundant as the healing process proceeds. Due to the degradability in the (human) body, magnesium and magnesium alloys qualify as a resorbable biomaterial. A porous structure enables easier interaction and access as well as the possibility to charge the implant with (bio-active) substances in order to facilitate the tissue to attach and grow into the implant, stimulate vascularisation, prevent inflammations and so on. In traumatology, for instance, such structures may be introduced for reconstruction of large bone defects: while the implant fixes and supports the bone, it gradually retracts and simultaneously stimulates the growth of new bone tissue at a similar pace. The process according to the invention can be used to control the morphology of the porous structure and hence the mechanical and desorption characteristics of the implant by the selection of appropriate metal powders (size, shape and their distribution), blending ratios (fractions of metal and polymer) and sintering parameters (temperature, time).

The following is a detailed description of certain preferred embodiments of the invention, which are presently deemed to be the best mode of carrying out the invention.

The proposed process involves combining fine magnesium or magnesium alloy powders with a polymer binder which allows the metal to be injected into a mould using standard plastic injection moulding machines. After the part is moulded and before the binder is removed, the part is referred to as a "green part". The next step is to remove the binder with a solvent and/or a thermal process (debinding). The resulting metal part is sintered in another unit operation at temperatures high enough to bond the particles but not melt the metal. During this sintering step, the component shrinks in a reproducible and linear manner. Sintering is concluded once that a desired porosity level is reached.

The process according to the invention uses magnesium alloy powders or granules for metal injection moulding (MIM) in combination with a polymer binder and any third substances, wherein a subsequent sintering process targets the creation of a structure of a pre-defined porosity rather than a fully dense compound.

Preferred embodiments include metal injection moulding of relatively complex shapes, thereby taking full advantage of the MIM-process over the conventional powder-metallurgical (P/M) approach.

A further preferred embodiment includes metal injection moulding in a mould in which is placed an insert of a different metal, so as to manufacture a hybrid component with differentiated functionality (i.e. partly resorbable, partly non-resorbable).

Alternative embodiments also include metal injection moulding in a continuous fashion by moulding on a roller, so as to manufacture a sheet green part as a basis for porous magnesium sheet.

For the discrete as well as for the continuous variants of the invention, a dynamic mould is devised so as to proceed with an in-mould sintering operation in which the parts can be calibrated to (near)-net shape.

As the mixing of the metal and polymer powders is a recognized bottleneck, a chaotic mixing process is proposed to improve product homogeneity.

A conventional Metal Injection Moulding (MIM) process involves combining fine metal powders (iron, copper, aluminium, tin, nickel, titanium, or refractory metals) with a polymer binder which allows the metal to be injected into a mould using standard plastic injection moulding machines. As thermoplastic/polymeric binders, several options are available. In principle, any polymers that are presently used for metal powder injection moulding qualify. No carbon residue should preferably be present in the green compact.

A suitable binder is polypropylene (PP) mixed with a wax, possibly paraffin in equal amounts. PP is cheap, available at high purity and in several grades. Other possible high molecular weight compounds comprise: polyethylene, methacrylate ester copolymers, ethylene-vinyl acetate copolymer, etc. Suitable waxes (low molecular weight compounds) include paraffin wax, carnauba wax, etc.

The selected PP should be dense (viscous), possibly having a melt flow index of 2-5. Since paraffin is very thin, the mixture will have an injection moldable viscosity. A binder mix that is too thin will let the powder sink. Here powder sinking is not as crucial as with steel/iron injection molding since Mg has a density of 1.8 $g/cm^3$ whereas ferrous alloys have a density of 7.8 $g/cm^3$. Many examples of binder compositions are also given in US 6,051,184.

Alternatively, the binder is a biodegradable polymer, for instance one of poly-L-lactic acid (PLLA), polyglycolic acid (PGA), poly-D,L-lactide.glycolide copolymer (PDLA), polyaprolactine (PCL) or the like. PLLA has a crystallinity of around 37%, a glass transition temperature between 50-80 °C and a melting temperature between 173-178 °C. Polyglycolide has a glass transition temperature between 35-40 °C and its melting point is reported to be in the range of 225-230 °C. The decomposition temperature of pure PLLA at infinite molecular weight is 353 °C. PLLA-Ca has a lower pyrolysis temperature (220-360 °C) and PGA has a pyrolysis temperature of 485 °C. After the part is moulded and before the binder is removed, the part or preform is referred to as a "green part". The next step is to remove the binder with a solvent and/or a thermal process (debinding). First the wax will be removed by leaching, and then the polymer will evaporate during sintering.

As regards binder removing solvents (i.e., for removing the wax parts of the binder), all sorts of hydrocarbon solvents would do: toluene, xylene, petroleum, white spirit, etc. Toluene can dissolve the wax at room temperature. Hexane heated at 50 °C for 6 h, removes wax resulting in an open porosity structure. Temperatures for thermally removing the binder (i.e., for removing polymer parts of the binder) can be achieved by pyrolysis of PP > 300-350 °C. Pyrolysis is the chemical decomposition of organic materials by heating in the absence of oxygen or any other reagents. Thus an inert environment for Mg does not affect the binder removal process. To transport the flammable gases produced during binder removal, the furnace chamber should be funnelled to the outside, or to some kind of gas receptacle. Biodegradable polymers do not need to be removed from the green part. In principle they might be removed during sintering, possibly the sintering temperature is well above decomposition temp of these polymers.

A third substance, i.e. HA (hydroxyl-apatite), might be introduced to the blend that is left behind after debinding.

The resulting metal part is sintered in yet a different unit operation at temperatures high enough to bond the particles but not melt the metal. For sintering a metallurgical furnace with controllable atmosphere (argon blowing, or other inert gas) may be employed. During this sintering step, the component shrinks in a reproducible and linear manner.

Sintering parameters, such as temperature, time, pressure and/or inert atmosphere that are particularly suitable for reactive magnesium or alloys thereof are generally known to the skilled person. A common shielding gas for liquid Mg contains $SF_6$. An argon gas environment is also suitable for Mg processing. Wen et al, Scripta Mater 45:1147-1153, 2001 -which relates to spacer foaming - provides sintering parameters that apply. 100MPa cold compaction, 200°C for 1hr (removing spacer), 500°C for 2h (sintering). Kikuchi et al, Mater Sci Forum, 39:475-479, 2005 - specifies hot pressing with 60 MPa at 350°C for 10 min. (for diffusion bonding). P.Burke and G.J. Kipouros of the Dalhousie University of Halifax, in a presentation of March 3, 2007 entitled: Development of Magnesium Powder Metallurgy Alloys, have analysed with design of experiments (DOE) principles several combinations of compaction pressure, sintering temperature, sintering time and quench temperature. The combinations studied include: 300-400-500MPa compaction, 500-550-600°C sintering for 20, 40, and 60 minutes, and quenched to 375 and 450°C.

The process according to the invention can be used to control the morphology of the porous structure and hence the mechanical and desorption characteristics of the implant by the selection of appropriate metal powders (size, shape and their distribution), blending ratios (fractions of metal and polymer) and sintering parameters (temperature, time). Ranges of porosity and pore size are typically those targeted for biomedical implants (relative density of 60-100% and opening size of 0.1-1 mm).

With respect to the relative amounts (or ranges thereof) of magnesium and binder powders in the powder mixture in volume and/or weight percentages (blending ratios; fractions), the following is to be observed. In the Metal Injection Moulding (MIM) feedstock, a standard volume ratio of metal powder to binder is 60-65% metal powder to 40-35% binder. This is an indicative standard ratio, which is not intended to be restrictive. [ADVANCED MATERIAL & PROCESSES, March 2005, pp 39-40]

It is important to observe the particle sizes and size distribution of the powders in the powder mixture and ranges thereof. In order to achieve an adequate performance concerning permeability, powders with a narrow range of particle size should be used. Apparent density of the selected powder can be adjusted by the particle size, shape and distribution.

The relevant background theory for Specific surface area ($S_m$) is a material property of solids which measures the total surface area per unit of mass, solid or bulk volume, or cross-sectional area. It is a derived scientific value that can be used to determine the type and properties of a material (e.g. soil). It is defined by surface area divided by mass (with units of $m^2/kg$) in the current context.

For monodispersive spherical particles:

$$S_m = 6/\left(\rho_t \phi\right)$$

For a distribution with n size classes (spherical):

$$S_m = \left(6/\rho_t\right)\left(\sum_{i=1}^{n}\phi_i^2 N_i\right)/\left(\sum_{i=1}^{n}\phi_i^3 N_i\right)$$

Where ρt is theoretical density, Ø is particle diameter and $N_i$ is the number of particles in each class. These values refer to a green compact, prior to sintering. However, $S_m$ can be related to the apparent density after sintering:

$$\rho = \frac{K}{S_m}$$   where ρ is the apparent density and K is an empirical constant.

Typical powder particle sizes for use in MIM processes are: 3 to 100 microns average diameter. Targeted pore sizes will be about 0.1 mm (100 microns - or less).

The process according to the invention can thus be used to process different magnesium alloy compositions and hence to tune the mechanical and desorption characteristics of the implant by the selection of the powder alloys and powder mixtures. This concerns the presence and amounts of the major and minor alloying elements (aluminium, zinc, manganese, or the like) as well as of any trace elements that have a pronounced influence on corrosion rate. Generally biodegradability of magnesium alloys in the human body will be at too fast a rate for implants. Accordingly alloys with an increased corrosion resistance will be preferred. With porous structures the problem of a too fast corrosion rate would also be further aggravated by the increased surface area. Alloy or trace elements, such as copper, nickel, and iron are therefore best avoided. A further consideration is that the alloying elements need to be bio-compatible (toxicity).

Particularly suitable magnesium alloys, as regards to bio-compatibility, corrosion rate, strength, toughness, oxide on powder for sinterability, comprise: Magnesium, magnesium alloys with Al, Ca, F, Li, Mg, Mn, rare earth elements, Zn, Zr, or the like or any combination thereof. Amongst the alloying elements mentioned above, only Zr can be considered as not bio-compatible, however Zr is used in very small amounts (0.1wt%) of the implant weight. A specific listing of elements for biomedical applications, as to their allowed amounts, lethal doses or like, is not available in the literature.

Aluminium (Al) in a content of more than 5 wt% is known to increase the corrosion resistance of Mg alloys significantly.

Rare-earth elements, Zr and Al all contribute to mechanical properties at room temperature and elevated temperatures by different mechanisms. Zinc (Zn) and Zirconium (Zr) contribute to ductility and toughness, by different mechanisms.

The natural oxide on magnesium is MgO.

## Pilling-Bedworth ratio (PB ratio)   R = Vmetaloxideproduced / Vmetalconsumed

When R <1, a metal oxide tends to be porous and non-protective, because it cannot cover the metal surface. R for Mg is 0.81. R is considered generally in the context of corrosion resistance, however it can also be used as an indicator for powder sintering. Since the powder surface is not fully covered with oxide, sintering is theoretically possible.

The process according to the invention can be used to manufacture finished parts that are (near-to)-net shape and of complicated geometry, but variants that target semi-finished parts (designed for tailoring by follow-up operations) and hybrid parts (assemblies in conjunction with non-resorbing parts) are conceivable as well.

The process according to the invention can be used to combine the two thermal processes after moulding into a single unit operation by using a polymer with a debinding temperature in the vicinity of the sintering temperature (between 350 and 550 °C for magnesium and magnesium alloys).

The process according to the invention can be used with a biodegradable polymer as the fluid medium, so that any remnants in the metal structure after binder removal are no biomedical concern. Also, a certain amount of polymer may be intentionally left in the metal structure to make a compound with mechanical characteristics close to that of the supported tissue.

The process according to the invention can be used with a polymer binder that is charged with (bio-active) substances that are left behind in the metal structure after debinding. This may serve as a corrosion inhibitor and/or to add other functionality. For instance, hydroxyl-apatite $Ca_{10}(PO_4)_6(OH)_2$ is known to be stable up to temperatures of 800 °C and suggested to act osteo-inductive; hence, its a candidate for incorporation as a stimulator of bone growth.

Drugs that do not disintegrate at temperatures up to the required sintering temperature may also include certain immuno-suppressive drugs and certain anti-inflammatory agents. General examples to immuno-suppressive drugs include: Analogues of rapamycin, such as tacrolimus (FK-506), sirolimus and more so everolimus, normally used as immuno-suppressants. As another approach, Morpholino oligos are used in Antisense knockdown of c-myc. Other medication against restenosis: paclitaxel, abraxane®, docetaxel, etc. Anti-inflammatory agents: Glucocorticoids, betamethasone, dexamethasone, prednisolone, corticosterone, budesonide, estrogen, sulfasalazine, mesalamine (see also: US 6,517,888B1).

Little or no information is available on thermal stability of rapamycin or taxol (paclitaxel). The only figures available show melting points of approximately 185 °C and 200-225 °C, respectively. So at those temperatures these are still intact. The sintering temperatures for magnesium alloys (especially above 450 °C) should be considered quite extreme for most bio-active molecules. Even without oxygen present most organic compounds do decompose without burning. If the compounds only melt and solidify again, there should not be a problem regarding bioactivity. Still it would be recommendable to determine the bioactivity of selected drug in a suitable cell system before and after heat treatment.

The drugs are released naturally in time from a top coating. There are 'drug release rate' curves in the literature.

a- closed structure: drugs or the like (HA) included during manufacturing, released during degradation. Thus must withstand sintering temperature (at least up to 550 °C)

b- open structure: drugs are included afterwards, in or out the surface. Sintering temperature should not present a problem in this regard. The process according to the invention can be used in conjunction with a subsequent coating operation so as to further tune the desorption time and/or add other functionality to the open porous structure. For instance, a plasma electric oxidation (PEO) process known as the Keronite process is suggested to be able to penetrate the pores and provide the internal surfaces with a corrosion-retarding coating as well as to be able to be charged with bio-active substances like hydroxyl-apatite.

A primarily preferred embodiment of the invention includes metal injection moulding of relatively complex shapes, thereby taking full advantage of the process over the conventional powder-metallurgical (P/M) route. A further preferred embodiment comprises of metal injection moulding in a mould in which an insert is placed of a different metal, so as to manufacture hybrid components with differentiated functionality (partly resorbable, partly non-resorbable). Inserts for hybrid structures of a different metal can include noble metals, such as: Ti alloys (Ti-6Al-4V), Co-Cr-Mo, AISI316L, Ta-based, or biodegradable metals, such as: pure Fe and Fe-Mn alloys , other Mg alloys or pure Mg.

An alternative embodiment includes metal injection moulding in a continuous fashion by moulding on a roller, so as to manufacture a sheet green part as a basis for porous magnesium sheet. Continuous molding and sintering porous magnesium sheet can be practised using commercially available injection molding equipment ('molding machines'). The components of the known injection molding machine include a plasticating unit, a clamping unit, control unit and a mold. A typical molding machine can be a single screw injection molding machine. For the discrete as well as for the continuous variant of the invention, a dynamic mould can be devised so as to proceed with an in-mould sintering operation in which the parts can be calibrated to (near)-net shape. As the mixing of the metal and polymer powders is a recognized bottleneck, a chaotic mixing process may be used to improve product homogeneity.

The steps perceived as more critical include mixing of magnesium powder in a polymer, plasticising and injecting magnesium and polymer-slurry into an injection moulding machine and the sintering of magnesium powder. Basically there are three categories of substances:

a first substance is magnesium alloy, which provides the porous structure;
a second substance is the binder (polymeric with additives); and
a third substance is formed by additives such as hydroxyl-apatite (HA, which stimulates bone growth). This last category can be added on a number of occasions during production: at mixing of the powders (then they must remain stable up to approximately 600 °C such as HA, contained in a possible coating which is applied to, or in, the porous structure, or after the porous product itself is finished (i.e. by immersion). Conceivably anti-inflammatory drugs that disintegrate at higher temperatures can be added. The agents or binder (second substance) must be removed preferably entirely, but possibly might remain in enclosed pores and these should not give problems when the implant dissolves. The invention also envisions that to the binder compositions are added, which upon removing of the binder deposit on the internal surface of the porous structure and may reduce corrosion.

Technical benefits include a manufacturing process for series production of resorbable biomedical implants that is efficient and flexible. There is an additional reduction of cycle time and costs when debinding and sintering can be integrated. An improved control over the morphology of the porous structure results in a better product performance. An increase in functionality can be obtained when any supplementary substances are included.

Biomedical devices, such as resorbable (bio-active) biomedical implants, including bone screws, dental implants, spinal cages, hip and knee implants can provide user and societal benefits. Quality of life can be enhanced by reduction in patient discomfort associated with surgery to remove temporary implants. This is achieved by redundancy of multiple interventions - including the associated operating risk and (general) anaesthesia - and because of faster healing. Healthcare and other costs will be reduced by a reduction in the number of surgeries and in hospital residence time, as well as less revalidation care and sick leave.

It is thus believed that the operation and construction of the present invention will be apparent from the foregoing description. The invention is not limited to any embodiment herein described and, within the purview of the skilled person; modifications are possible which should be considered within the scope of the appended claims. Equally all kinematic inversions are considered inherently disclosed and to be within the scope of the present invention. While the specification in general may refer to magnesium or its alloys, it is to be understood that in practise the magnesium used will mostly be alloyed. The term comprising when used in this description or the appended claims should not be construed in an exclusive or exhaustive sense but rather in an inclusive sense. Expressions such as: "means for ..." should be read as: "component configured for ..." or "member constructed to ..." and should be construed to include equivalents for the structures disclosed. The use of expressions like: "critical", "preferred", "especially preferred" etc. is not intended to limit the invention. Features which are not specifically or explicitly described or claimed may be additionally included in the structure according to the present invention without deviating from its scope.

## Claims

1. Method of manufacturing a porous metal or metal alloy structure having a redefined porosity, the method including:

   mixing predefined amounts of a metal or metal alloy powder and a binder powder into a flowable powder mixture;
   providing a mould and injecting the flowable powder mixture into the mould to obtain a preform moulded into a desired shape;
   allowing solidification of at least the binder;
   removing the binder from the preform to obtain a substantially binder-free preform;
   sintering the substantially binder-free preform during a time and at a temperature to achieve the predefined porosity, and wherein the mixing is performed using metal or metal alloy powder that includes magnesium or at least one of its alloys.

2. Method according to claim 1, wherein the binder is a polymeric material.

3. Method according to claim 2, wherein the binder is a thermoplastic binder.

4. Method according to claim 3, wherein the polymeric binder is a high molecular weight compound.

5. Method according to claim 3 or 4, wherein the polymeric binder is polypropylene (PP).

6. Method according to claim 4, wherein high molecular weight compound is selected from a group comprising polyethylene, methacrylate ester copolymers, and ethylene-vinyl acetate copolymer.

7. Method according to any of the preceding claims, wherein the binder is a biodegradable polymer.

8. Method according to claim 7, wherein the biodegradable polymer is selected from a group comprising poly-L-lactic (PLLA), polyglycolic acid (PGA), poly DL-lactide (PDLA), poly DL-lactide/glycolide (PGLA), and polyprolactine (PCL).

9. Method according to any preceding claim, wherein the binder is mixed with wax.

10. Method according to claim 9, wherein the wax is selected from a group comprising paraffin wax, carnauba wax, and other low molecular weight compounds.

11. Method according to any preceding claim, wherein the metal or metal alloy structure is a medical appliance.

12. Method according to claim 11, wherein the medical appliance is a biomedical implant.

13. Method according to any preceding claim, wherein the binder is removed with a solvent, preferably by solvent immersion.

14. Method according to any preceding claim, wherein the binder is removed thermally.

15. Method according to claim 14, wherein the moulded preform is heated gradually in one of a controlled atmosphere and vacuum to allow the binder to evaporate.

16. Method according to claim 15, wherein the controlled atmosphere is an inert environment and wherein the binder is chemically decomposed at a temperature between 300 and 350 °C.

17. Method according to any preceding claim, wherein the metal or metal alloy structure is further provided with at least one substance that adds a predetermined functionality thereto.

18. Method according to claim 17, wherein at the mixing step the powder mixture is charged with the at least one substance, as an additive that is left behind in the metal structure after debinding.

19. Method according to claim 17 or 18, wherein a polymer binder is charged with at least one, preferably bio-active, substance that is left behind in the metal structure after debinding.

20. Method according to claim 18 or 19, wherein the at least one substance remains stable up to approximately 600 °C.

21. Method according to claim 19 or 20, wherein the at least one substance is hydroxyl-apatite (HA).

22. Method according to claim 17, wherein the at least one substance is contained in a coating, which is applied to, or in, the porous metal or metal alloy structure.

23. Method according to claim 17, wherein the at least one substance is added after the porous structure itself is finished.

24. Method according to claim 23, wherein the at least one substance is added by immersion of the finished porous structure.

25. Method according to any of the preceding claims, wherein anti-inflammatory drugs are added that disintegrate at higher temperatures.

26. Method according to any one of claims 17 to 24, wherein the at least one substance is a bio-active substance.

27. Method according to claim 26, wherein the bio-active substance includes an anti-inflammatory agent.

28. Method according to claim 27, wherein the anti-inflammatory agent is selected from a group comprising glucocorticoids, betamethasone, dexamethasone, prednisolone, corticosterone, budesonide, estrogen, sulfasalazine, and mesalamine.

29. Method according to claim 26, wherein the bio-active substance includes an immuno-suppressive drug.

30. Method according to claim 29, wherein the immuno-suppressive drug is selected from a group comprising analogues of rapamycin, including tacrolimus (FK-506), sirolimus and preferably everolimus.

31. Method according to claim 29, wherein the immuno-suppressive drug is selected from a group comprising morpholino oligos, used in antisense knockdown of c-myc, other medication against restenosis, paclitaxel, abraxane®, and docetaxel.

32. Method according to any one of claims 26 to 31, wherein the bio-active substance includes hydroxyl-apatite.

33. Method according to any of the preceding claims, wherein morphology of the porous metal or metal alloy structure is controlled so as to provide mechanical and desorption properties useful for its use as an implant.

34. Method according to claim 33, wherein the morphology is controlled by appropriate selection of metal powders, as to their size, shape and distribution.

35. Method according to claim 34, wherein powders for the powder mixture are selected with a narrow range of particle sizes.

36. Method according to claim 35, wherein the particle size is selected to be between 3 to 100 microns.

37. Method according to claim 33 or 34, wherein morphology is controlled by appropriate selection of blending ratios, as to fractions of metal and polymer.

38. Method according to claim 37, wherein the metal powder is present in a ratio of 60-65% by volume and the binder powder in a ratio of 40-35% by volume.

39. Method according to any one of claims 33 to 38, wherein morphology is controlled by appropriate selection of sintering parameters, as to temperature, and time.

40. Method according to claim 39, wherein sintering is effected at a temperature between 350 and 550 °C, for a duration of between 20 and 60 minutes.

41. Method according to any of the preceding claims, wherein ranges of porosity and pore size are targeted for biomedical implants and comprise a relative density of 60 to 100% and a pore size of 0.1 mm or less.

42. Method according to any of the preceding claims, wherein mechanical and desorption properties are tuned by selecting amongst different magnesium alloy compositions for powder alloys and powder mixtures.

43. Method according to claim 42, wherein tuning of the mechanical and desorption properties includes presence and amount of major and minor alloying elements, preferably including any one of aluminium, zinc, and manganese.

44. Method according to any of the preceding claims, wherein alloying elements are selected to be bio-compatible.

45. Method according to any of the preceding claims, wherein the steps of binder removal after moulding and sintering are combined into a single thermal operation by using a binder having a debinding temperature in the vicinity of the sintering temperature.

46. Method according to claim 45, wherein the debinding temperature of the binder ranges from about 350 to 550 °C.

47. Method according to any of the preceding claims, wherein a given amount of binder is intentionally left in the metal or metal alloy structure to obtain predefined mechanical properties.

48. Method according to claim 21 or 32, wherein the at least one substance serves as a corrosion inhibitor.

49. Method according to claim 31, 32 or 48, wherein the at least one substance serves to add a different functionality.

50. Method according to claim 21, 32, 48 or 49, wherein the at least one substance serves as a stimulator of bone growth.

51. Method according to claim 49 or 50, wherein the at least one substance is hydroxyl-apatite: $Ca_{10}(PO_4)_6(OH)_2$.

52. Method according to any preceding claim further including a subsequent coating step so as to provide any one of a desorption time and addition of other functionality to the open porous metal or metal alloy structure.

53. Method according to claim 52, wherein a plasma electric oxidation (PEO) step, known as Keronite process, is used to enable penetration of pores and provide internal surfaces thereof with any one of a corrosion-retarding coating and a bio-active substances, such as preferably hydroxyl-apatite.

54. Method according to any of the preceding claims, wherein prior to the step of injecting, an insert of a different metal is positioned in the mould to manufacture a hybrid structure offering differentiated functionalities.

55. Method according to claim 54, wherein the hybrid structure is partly resorbable and partly non-resorbable.

56. Method according to claim 54 or 55, wherein the insert is of a metal or metal alloy selected from a group including Titanium alloys, such as Ti-6Al-4V, Co-Cr-Mo, AISI316L, Ta-based, pure iron (Fe), Fe-Mn alloys, Mg alloys and pure Mg.

57. Method according to any of the preceding claims, which includes metal injection moulding in a continuous fashion by moulding on a roller, so as to manufacture a sheet-like preform as a basis for a porous magnesium or magnesium alloy sheet.

58. Method according to any of the preceding claims, wherein the mixing of the magnesium based powders and the binder powders uses a chaotic mixing process for improving product homogeneity.

**Europäisches Patentamt**
**European Patent Office**
**Office européen des brevets**

# EUROPEAN SEARCH REPORT

Application Number

EP 08 16 1449

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 604 919 A (STERZEL HANS-JOSEF [DE] ET AL) 18 February 1997 (1997-02-18) * abstract * * column 1, line 6 - line 19 * * column 2, line 15 - line 41 * * column 3, line 1 - line 54; example 1 * ----- | 1-6,9, 10,13,45 | INV. B22F3/11 C22C1/08 A61L27/04 A61L27/42 A61L27/54 A61L27/56 |
| X | US 5 064 463 A (CIOMEK MICHAEL A [US]) 12 November 1991 (1991-11-12) <br><br> * column 1, line 6 - line 10 * * column 2, line 18 - column 4, line 8 * * column 5, line 7 - line 18 * * claims 1-11; example * ----- | 1-6,9, 10, 13-16, 45,46,58 | |
| X | WO 03/076109 A (ADVANCED CERAMICS RES INC [US]; ARTZ GREGORY [US]; VAIDYANATHAN RANJI) 18 September 2003 (2003-09-18) * page 3, last paragraph - page 4, paragraph 5 * * page 6, paragraph 3 - page 7, last paragraph * * page 9, paragraph 2 - page 10, paragraph 2 * ----- | 1-4,6,9, 10,14,58 | |
| X | WO 2008/087213 A (CINV AG [DE]; ASGARI SOHEIL [DE]) 24 July 2008 (2008-07-24) <br><br> * abstract * * page 2, line 24 - page 6, line 23 * * page 7, line 20 - page 15, line 23 * * page 18, line 26 - page 20, line 21 * * examples * ----- | 1-6,11, 12, 14-16, 45,46 | |

TECHNICAL FIELDS
SEARCHED (IPC)

B22F
C22C
A61L

~~The present search report has been drawn up for all claims~~

| Place of search | Date of completion of the search | Examiner |
|---|---|---|
| The Hague | 20 March 2009 | Ceulemans, Judy |

Europäisches
Patentamt

European
Patent Office

Office européen
des brevets

Application Number

EP 08 16 1449

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6, 9-16, 45-46, 58

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 08 16 1449

The Search Division considers that the present European patent application does not comply with the requirements of unity of invention and relates to several inventions or groups of inventions, namely:

1. claims: 1-6, 9-16, 45-46,58

   Method of making a porous metal (alloy) structure by means of injection moulding a mixture of a metal powder and a binder, removing the binder from the preform and sintering the binder-free preform, wherein Mg (alloy) is included in the metal powder
   ---

2. claims: 7-8

   method of making a porous Mg (alloy) structure by means of injection moulding a Mg (alloy) powder and a binder and sintering the preform after dissolving the binder, wherein the binder is biodegradable.
   ---

3. claims: 18-24

   method of making a porous Mg (alloy) structure by means of injection moulding a Mg (alloy) powder and a binder and sintering the preform after dissolving the binder, wherein a further substance is added for a further functionality to the structure be it by addition to the mixture or application before or after the sintering of the structure ;
   ---

4. claims: 25-32, 48-51

   method of making a porous Mg (alloy) structure by means of injection moulding a Mg (alloy) powder and a binder and sintering the preform after dissolving the binder, wherein a further substance is added for a further functionality to the structure by the addition of specific agents or drugs
   ---

5. claims: 33-44, 47

   method of making a porous Mg (alloy) structure by means of injection moulding a Mg (alloy) powder and a binder and sintering the preform after dissolving the binder, whereby the morphology of the structure is controlled to provide mechanical and desorption properties for it to be useful as an implant.
   ---

6. claims: 52-53

Europäisches
Patentamt

European
Patent Office

Office européen
des brevets

**LACK OF UNITY OF INVENTION
SHEET B**

Application Number

EP 08 16 1449

The Search Division considers that the present European patent application does not comply with the
requirements of unity of invention and relates to several inventions or groups of inventions, namely:

method of making a porous Mg (alloy) structure by means of
injection moulding a Mg (alloy) powder and a binder and
sintering the preform after dissolving the binder, wherein
the structure is treated with a plasma electric oxidation
step

---

7. claims: 54-56

method of making a porous Mg (alloy) structure by means of
injection moulding a Mg (alloy) powder and a binder and
sintering the preform after dissolving the binder, wherein
an insert of a different metal is added

---

8. claim: 57

method of making a porous Mg (alloy) structure by means of
injection moulding a Mg (alloy) powder and a binder and
sintering the preform after dissolving the binder, whereby
the moulding is performed on a roller to obtain a sheet-like
preform.

---

**ANNEX TO THE EUROPEAN SEARCH REPORT
ON EUROPEAN PATENT APPLICATION NO.**                    EP 08 16 1449

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2009

| Patent document cited in search report | | Publication date | Patent family member(s) | | Publication date |
|---|---|---|---|---|---|
| US 5604919 | A | 18-02-1997 | NONE | | |
| US 5064463 | A | 12-11-1991 | NONE | | |
| WO 03076109 | A | 18-09-2003 | AU | 2003228289 A1 | 22-09-2003 |
| WO 2008087213 | A | 24-07-2008 | US | 2008175885 A1 | 24-07-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6725901 A
- US 20060271168 A
- US 6641776 B
- WO 2007068479 A
- US 6051184 A
- US 6517888 B1

**Non-patent literature cited in the description**

- **Banhart J.** Manufacture, characterisation and application of cellular metals and metal foams. *Progress in Materials Science,* 2001, vol. 46, 559-632
- **Wen et al.** *Scripta Mater,* 2001, vol. 45, 1147-1153
- **Kikuchi et al.** *Mater Sci Forum,* 2005, vol. 39, 475-479
- **P.Burke ; G.J. Kipouros.** *Development of Magnesium Powder Metallurgy Alloys,* 03 March 2007
- *ADVANCED MATERIAL & PROCESSES,* March 2005, 39-40